# EUROPEAN PATENT SPECIFICATION

(11) **EP 3 462 457 B1**
(45) Date of publication and mention of the grant of the patent: **19.05.2021**
(21) Application number: 18187339.9
(22) Date of filing: 24.04.2014
(51) Int. Cl.: G16H 10/60, G16H 40/20, G16H 40/67

(54) **SECURITY SYSTEM WITH PORTABLE MEDICAL DEVICE**
SICHERHEITSSYSTEM MIT TRAGBARER MEDIZINISCHER VORRICHTUNG
SYSTÈME DE SÉCURITÉ COMPORTANT UN DISPOSITIF MÉDICAL PORTABLE

(30) Priority: 26.04.2013 US 201361816636 P; 04.02.2014 US 201414172585
(43) Date of publication of application: 03.04.2019
(62) Divisional of application: 14727716.4
(73) Proprietor: Roche Diabetes Care GmbH, 68305 Mannheim (DE); F. Hoffmann-La Roche AG, 4070 Basel (CH)
(72) Inventor: CARLSGAARD, Erik, Zionsville, 46077 (US); DINWIDDIE, Aaron, Cicero, 46034 (US); GEJDOS, Igor, Indianapolis, 46240 (US); LONG, Michael Lee, Pendleton, 46064 (US); OELMANN, Teresa, Noblesville, 46060 (US)
(74) Representative: Richardt Patentanwälte PartG mbB

(56) References cited:
- US-A1- 2001 032 099
- US-A1- 2007 100 222
- US-A1- 2008 071 580
- US-A1- 2010 223 184
- Anonymous: "Datenmüll-Management - Wikipedia", , 9 March 2013 (2013-03-09), XP055153091, Retrieved from the Internet: URL:http://de.wikipedia.org/w/index.php?ti tle=Datenm%C3%BCll-Management&oldid=115174 192 [retrieved on 2014-11-14]
- "Getting Started - CareLink Personal", , 1 January 2011 (2011-01-01), pages 1-11, XP055081464, North Ryde, NSW, Australia Retrieved from the Internet: URL:http://www.medtronic-diabetes.com.au/w cm/groups/mdtcom_sg/@mdt/@ap/@au/@diabetes /documents/documents/contrib_107976.pdf [retrieved on 2013-09-27]

## Description

### CROSS-REFERENCE TO RELATED APPLICATIONS

This application claims the benefit of U.S. Provisional Application No. 61/816,636, filed on April 26, 2013.

### FIELD

The present disclosure relates to portal control features that pertain to a diabetes management system.

### BACKGROUND

This section provides background information related to the present disclosure which is not necessarily prior art.

Persons with diabetes often have difficulty regulating blood glucose levels in their bodies. As a consequence, many of these persons carry specialized electronic meters, called blood glucose meters, which allow them to periodically measure their glucose levels and take appropriate action, such as administering insulin. After a blood glucose measurement or series of measurements is taken, a diabetic patient may find it useful to communicate these measurements to his or her healthcare professional for further review and analysis. In this regard, the patient's blood glucose meter may be capable of storing the blood glucose measurements for later review and analysis by the patient or the healthcare professional, who may then record the measurements manually or electronically.

The process of measuring, storing, recording and analyzing blood glucose levels can be a very time consuming process for both the patient and the patient's healthcare professional. Often, the exchange and review of data requires a meeting between the patient and the healthcare professional. People with diabetes are often searching for better and more efficient ways to manage their health. In addition, healthcare professionals need new tools to motivate people with diabetes to communicate more effectively. Technology can provide a viable platform for software applications for a wide variety of consumer demands. Moreover, many people with diabetes use personal computers and/or mobile devices in their daily lives.

Patent application US 2010/223184 A1 describes systems and methods for primary and sponsored accounts. The system comprises an account processor to execute software instructions for creating and managing electronic payment accounts and an accounts database to store accounts data from the account processor. The account processor may be configured to create a primary account and a sponsored account in the accounts database. The primary account may be associated with a primary account holder who has access to the primary account to add and remove funds. The sponsored accounts may be associated with both the primary account holder and a sponsored account holder, where the primary account holder has access to the sponsored account to transfer funds between the primary account and the sponsored account in order to add and remove funds from the sponsored account, and the sponsored account holder has access to the sponsored account for making transactions using funds in the sponsored account.
US patent application US 2007/100222 A1 describes a system for monitoring blood glucose data of a patient. The system includes a sensing device and hospital monitor. The sensing device includes a sensor and sensor electronics and is adapted to transmit information to the hospital monitor while continuing to sense blood glucose data. The communication between the sensing device and the hospital monitor may be wireless. The sensor electronics may include a sends a power supply, a voltage regulator, and optionally a memory and processor.

US patent application US 2008/071580 A1 describes a diabetes management system (DDMS) and corresponding method for assisting a healthcare professional and monitoring and evaluating the progress of a diabetic patient by generating various types of reports that are indicative of periodic trends in the patient's behavior, including compliance with a prescribed course of therapy. Specifically, input data, including carbohydrate, insulin, and glucose data are uploaded by the patient into the DDMS, which then generates periodic, e.g. weekly, patient-specific output data in the form of box plot graphs, bar graphs, etc. over an extended period of time.

The German Wikipedia entry for "Datenmüll-Management" of March 9, 2013, retrieved from the Internet http://de.wikipedia.orq/w/index.php?title=Datenm% C3%BCII-Management&oldid= 115174192 [retrieved on 2014-11-14] gives an overview on various strategies on how to handle redundant or outdated data by companies and individual persons.

An article "getting started-CareLink Personal", 1st January 2011, pages 1-11, XP055081464, North Ryde, NSW, Australia, retrieved from the Internet: URL: http://www.medtronic-diabetes.conn.au/wcnn/qroups/mdtconn sg/@mdt/@ap/@au/ @diabetes/documents/documents/contrib_107976.pdf, describes a free online tool that allows the discovery of patterns and problems that meter software and log-books may not uncover. In particular, it allows to understand the effects of insulin, carbohydrates and exercise on the glucose level and identify glucose patterns and problems. Data security issues are also addressed.

US patent application US 2001/032099 A1 describes an apparatus for providing healthcare information. The apparatus includes a memory device for storing information for performing a healthcare diagnosis, prescribing a treatment for a healthcare diagnosis, and/or for monitoring a treatment. The apparatus further comprises a receiver for receiving information regarding at least one of an individual's symptom, an individual's condition, and/or an individual's illness, wherein the information can be generated by a machine or a device. A processor determines a diagnosis and/or a prescribed treatment.

A problem involved with measuring blood glucose levels with portable glucose meters is that the management of health data is time consuming both for the patient and for the healthcare provider and that sensitive patient data may be visible to third parties if the glucose meter or a personal computer comprising sensitive patient data is lost or stolen.

### SUMMARY

This section provides a general summary of the disclosure, and is not a comprehensive disclosure of its full scope or all of its features.

The scope of the invention is defined by the claims. The embodiments of the invention can freely be combined with each other if they are not mutually exclusive.

In one aspect, not being part of the claimed invention, the disclosure relates to a security system for medical data
The examples, aspects and embodiments, if any, disclosed in the following description that do not fall within the scope of the claims are for reference only, and are to be interpreted as examples useful for understanding various embodiments of the invention.

The system comprises a portable medical device for measuring medical parameters which are indicative of a physiological state of a mammal, e.g., a patient.

The system further comprises a server device which comprises and/or which is operatively coupled to a storage device. The server device hosts an accounts manager of a diabetes management system. The accounts manager is configured to manage accounts stored on said storage device. Said accounts comprise a first account associated with a healthcare provider and a plurality of patient accounts associated with said first account. Each patient account comprises medical data of a respective patient.

The system further comprises a mobile device hosting an application program. Said application program is interoperable with the portable medical device and with the accounts manager. The mobile device is connected with the server device via a network. The mobile device may be, for example, a mobile phone, in particular a smart phone, a tabloid computer, a notebook or the like.

The portable medical device is configured to repeatedly measure medical parameters of one of said patients.

The application program is configured to repeatedly and fully automatically receive the measured medical parameters from said measurement device. The application program may be configured to immediately forward the received measured medical parameters to the accounts manager for storing said parameters on the storage device of the server as part of the patient account of said patient.

The portable medical device, the application program and the accounts manager are configured such that only a single copy of the measured medical data is permanently stored in said storage device and such that any temporally created copies of said measured medical data are immediately erased from the portable medical device and the mobile device after execution of the forwarding.

The account manager is configured to execute the following steps:
- receiving a request to deactivate a first account;
- removing access to data associated with the first account;
- identifying each of the patient accounts associated with the first account and deactivating each of the identified patient accounts;
- sending an electronic notification to the patients associated with each of the identified patient accounts, where the electronic notification advises the patient that the corresponding patient account has been deactivated;
- creating an audit record in a data store, where the audit record indicates the deactivation of the first account; and
- sending a termination command to the application programs of the patients associated with each of the identified patient accounts, wherein the application programs are configured such that they immediately terminate forwarding of medical measurement data to the accounts manager.

Said features may be advantageous for multiple reasons: that the measurement data is automatically forwarded and centrally stored by the server device may ease the collection and storing of health-related measurement data for the patient. A continuous collection of such data may be advantageous as said data may be used for diagnostic purposes and in order to determine an appropriate treatment schema and an appropriate amount of drug for treatment. As the forwarding is executed fully automatically, the patient is freed of the burden of submitting said data manually and the chances rise that the forwarded measurement data provides a complete documentation of the measured parameter over a longer period of time.

In a further beneficial aspect, only a single copy of the measured medical data is permanently stored in said storage device of the server. Neither the portable medical device nor the mobile device of the patient store a copy of the measurement data as said data is immediately erased after a successful forwarding. Any temporally created copies of said measured medical data, e.g. data kept in the main memory of the portable medical device or the mobile device, are immediately erased after the forwarding of the data to the server device. This ensures that even in case the portable medical device and/or the mobile device should be lost, an unauthorized person having found or having stolen said devices will not be able to obtain any measurement data from the device as the only existing copy is stored on a storage device of the server.

In a further beneficial aspect, the data is also protected in case a healthcare provider having assigned the first account has lost his access credentials, e.g. because his or her notebook having stored the access credentials to the first account have been stolen. In such an event, the healthcare provider merely needs to send a request for deactivating his account to the accounts manager. This request will also result in a deactivation of all patient accounts of patients said healthcare provider is responsible for. Thus, an automated, centralized and secure way of collecting and managing medical measurement data and accounts is provided.

In a further beneficial aspect, embodiments of the invention may improve the effectiveness and efficiency of storing, communicating and analyzing blood glucose measurements. Redundantly storing multiple copies of the data on different devices of the patient and the patient's healthcare professional may be avoided. Sending data, including blood glucose measurements, to a centralized electronic data repository for later retrieval and analysis may reduce the amount of consumed storage space and network traffic. In a further beneficial aspect, the centrally stored patient data is available for the patient and the patient's healthcare professional at various times and from various locations. The sharing of data and the data repository can be managed by a single set of collectively manageable administrative guidelines implemented e.g. by rules executed by the server device. Such administrative guidelines may need to follow certain procedures for opening, closing, and deactivating both patient and healthcare provider accounts associated with the data sharing and storage.

According to embodiments the portable medical device comprises a processor and an interface for exchanging data with the application program. The interface is capable of pairing the portable medical device to the application program. In a paired state, the portable medical device and the application program are connected via a data communication connection. Only in the paired state the one portable medical device is configured for enabling a transferring of measurement data to the application program. For example, the application program may execute the pairing in interoperation with the portable medical device if the application program determines by evaluating a device-ID of the portable medical device that said device has already successfully registered at the accounts manager.

Said features may be advantageous as they may ensure that the application program only forwards measurement data gathered by a portable medical device that has successfully paired with the application program and that thus can reliably be considered as trustworthy. For example, there may be medical devices on the market that do not fulfill certain quality standards defined by the accounts manager or that may not be used without prescription in a particular country. The accounts manager may refuse to register such kinds of medical devices. Said refusal may be communicated from the accounts manager to the application program. This will automatically result in an incapability of the portable medical device and the application program to establish a pairing and to forward the medical data via the application program to the accounts manager. This may help to protect the safety of the patient and to increase the quality of the measurement data stored and managed by the accounts manager.

According to embodiments, the connection established after pairing may be a WiFi connection, a Bluetooth connection, a cable connection, or any other suitable data transfer connection. Preferable, the connection is secured by a cryptographic protocol, e.g. SSH and/or TLS. If the pairing was executed successfully, the portable medical device and the application program are in a paired state. Otherwise, both objects are not in the paired state.

According to embodiments, the application program is configured to execute steps comprising:
- receiving a pairing command from the patient; the pairing command may be received from the user via a user interface, e.g. a GUI displayed on the mobile device, or from the portable medical device that may have some measurement data available; the receiving of the pairing command may, for example, be triggered by the patient who turns on the portable medical device in the proximity to his mobile device for executing a measuring; alternatively, the portable medical device may automatically execute the measuring regularly and may prepare executing a measuring by triggering the initiation of the pairing; alternatively, the pairing may be initiated by a portable medical device that has already gathered some measurement data and then tries to establish a pairing in order to forward the measurement data to the application program;
- in response to the receiving of the pairing command, requesting a device-ID from the portable medical device;
- in response to said request, receiving the device-ID from the portable medical device; in cases where the device cannot return a device-ID, said device-ID may be generated by the application program and assigned to said device, see below;
- evaluating the received device-ID for determining if a pairing between the application program and the medical measuring device has already been established at least once previously; for example, the determining may be executed by the application program by comparing the received device-ID with one or more device-IDs stored already by the application program on a storage of the mobile device;

For example, in case a pairing between the application program and the medical measuring device has already been established at least once previously, the storage medium of the mobile device may already have stored the device-ID in association with the patient's patient-ID and in association with a confirmation of the accounts manager that said portable medical device has successfully registered at the accounts manager. Said confirmation of the accounts manager may comprise a signature that can be verified by the application program by means of a certificate chain evaluation.
- only if it is determined that the pairing between the application program and the medical measuring device has already been established at least once previously, automatically establishing the pairing in interoperation with the portable medical device by creating the data communication connection; and automatically executing the forwarding of the measurement data; as described above, the measurement data may be forwarded without creating and storing any additional copies of the measurement data on the mobile device; the measurement data may immediately be deleted from a storage of the portable medical device after the forwarding;
- only if it is determined that the pairing between the application program and the medical measuring device has not yet been established at least once previously, automatically sending a device-registration request to the accounts manager, the device registration request comprising the received device-ID and a patient-ID of the patient using the mobile device; and only executing the forwarding of the measurement data after having received a confirmation from the accounts manager that the portable medical device was registered for said patient at the accounts manager successfully.

According to embodiments the application program is configured such that it executes the following steps:
- in response to receiving the confirmation from the accounts manager that the portable medical device was successfully registered for said patient at the accounts manager, permanently storing said device-ID in a storage medium of the mobile device as a reference value in association with the confirmation of the accounts manager; and
- in response to receiving a later pairing command, determining, by comparing the stored reference device-ID with a device-ID received from the medical measuring device, that a pairing between the application program and the medical measuring device has already been established.

Said features may be advantageous as they ensure that only trusted portable medical devices with known quality standards may be used to upload medical measurement data to the accounts manager. In a further aspect, the uploading is facilitated. Once the portable device has successfully registered at the accounts manager, it is not necessary that the application program and the accounts manager running on the server device exchange any data over the network to initiate the pairing. Rather, the pairing may be executed immediately and without exchanging data over the network if the mobile device already has stored the device-ID in association with a registration confirmation of said device for said particular patient.

According to embodiments the application program is configured such that receiving the device-ID from the portable medical device comprises:
- receiving a message from the portable medical device that no device-ID can be returned to the application program;
- automatically creating a random character string; for example, the random character string may comprise any combination of alphanumeric characters;
- returning the created random character string to the portable medical device for permanent storage in a storage of said portable medical device as a device-ID of said measurement device;
- using the created random character string as the received device-ID.

Said features may be advantageous as it is possible to make use of portable medical devices of a type that is not able to communicate a unique device-ID to the application program. By automatically generating a device-ID and storing said generated ID in a storage medium of the device, it is possible to provide the above described security system also based on device types originally lacking a suitable device-ID.

According to embodiments the accounts manager is configured such that successfully registering the portable medical device at the accounts manager comprises:
- authenticating the patient; for example, the patient may log- into the account manager by providing some authentication information such as username and password, biomedical data such as a fingerprint, a security token or the like;
- receiving a confirmation from the authenticated patient that the portable medical device identified by the device-ID in the device registration request shall be registered;
- if said patient's confirmation is received, storing the device-ID contained in the device-registration request by the accounts manager in association with the patient-ID in the device registration request, the patient-ID identifying the patient account of said patient.

This may be advantageous as the accounts manager may thus check and ensure that each registered portable medical device is assigned to one patient only. This may help to ensure that no sensitive medical data is assigned to and stored in the wrong patient account.

According to embodiments, the accounts manager is configured such that only measurement data collected by a portable medical device having successfully been registered at the accounts manager is received and stored in association with a respective patient account.

In addition or alternatively, the application program may be configured such that only measurement data collected by a portable medical device having successfully been registered at the accounts manager is forwarded to the accounts manager.
According to some embodiments, the device-ID of the portable medical device is a MAC address of the device, a serial number of the device, or any other data value that is unique for said portable medical device and that can be read out by the application program or that can otherwise be communicated from the portable medical device by the application program. In some embodiments, the portable medical device may not comprise such a device-ID. In these cases, the application program may generate a random data value upon initial registration of said device at the account manager and store said device-ID in a storage of said portable medical device and in a data store of the mobile device hosting the application program. In said cases, the generated random number may be used as the device-ID; the determination may involve the application program interacting with the portable medical device to execute the following steps:
- prompting a patient who may own or use the portable medical device to confirm the device-ID received from the portable medical device or generated de novo by the application program;
- in case the patient confirms, storing the determined device-ID in association with an accounts-ID of the patient's account in a permanent storage medium of the mobile device;

According to some embodiments, the user does not have to authenticate at the account manager by means of a password or fingerprint once his portable medical device was registered at the accounts manager. Rather, a device-ID of the portable medical device is sent together with the measurement data to the accounts manager. Said device-ID is used to assign the forwarded measurement data to the patient account of the patient owning or using said portable medical device. Thus, the forwarding of the measurement data is accelerated, facilitated and automated. Nevertheless, the method is save as the device registration process requires a user authentication at the account manager.

According to embodiments, the medical measuring device is a glucose meter. The measurement data may be a glucose level obtained by analyzing, for example, a blood sample, a urine sample or a saliva sample of the patient.

According to the invention the account manager is configured to execute, upon receiving the request to deactivate the first account, the steps of:
- disabling data synchronization functions, the data synchronization functions being functions configured to exchange data between a first and a second storage device, the exchanged data belonging to the first account and/or one of the identified patient accounts, the first storage device being part of and/or being operatively coupled to the server device, the second storage device being part of the medical measuring device; for example, the transfer of the measurement data from the second storage device of the portable medical device to a patients account stored in the first storage device being part of the server device may be executed by means of data synchronization functions which may immediately be followed by data erasing functions for erasing the measured data from the portable medical device and from the mobile device; and/or
- disabling data sharing functions, the data sharing functions being configured to exchange data between the first account and each of the identified patient accounts, wherein the data belonging to the first account and to the identified patient accounts both are stored on a storage device that is part of and/or is operatively coupled to the server device.

This may be advantageous as it is prohibited that a patient may forward sensitive patient data contained in his patient account to the first account of his health care provider in case it cannot be ensured that an unauthorized third party may have acquired access credentials of said health care provider.

The invention relates to a computer-implemented method for managing accounts in a diabetes management system, comprising:
- receiving, by an account manager, a request to deactivate a first account associated with a healthcare provider, where the first account is associated with a plurality of patient accounts and the account manager is implemented as computer executable instructions executing on a computer processor of a server device; the first account and the plurality of patient accounts and respectively associated data may be stored on a non-temporary, tangible storage medium that is part of or operatively coupled to the server device; for example, said accounts could be stored in a relational database hosted by the server device or a separate database server operatively coupled to the server device via a network connection;
- removing, by the account manager, access to data associated with the first account;
- identifying, by the account manager, each of the patient accounts associated with the first account and deactivating each of the identified patient accounts;
- sending, by the account manager, an electronic notification to a person associated with each of the identified patient accounts, where the electronic notification advises the person that the corresponding patient account has been deactivated; and
- creating, by the account manager, an audit record in a data store, where the audit record indicates the deactivation of the first account.

According to embodiments the method further comprises archiving data associated with each of the plurality of patient accounts for a predetermined period of time. According to some embodiments the predetermined period of time is associated with a geographic location for each respective patient. This may be advantageous as it allows to adapt the duration of said time period to various data protection requirements defined differently by different countries.

According to some embodiments the predetermined period of time can be associated with a regulatory compliance. According to some embodiments, the regulatory compliance is associated with a geographic location of the patient, e.g., a country that may be represented in a patient's account by a country code.

The invention further comprises disabling, by the account manager, data synchronization functions. Data synchronization functions are functions which are configured to exchange data between a first and a second storage device. The exchanged data may belong to the first account and/or to one of the identified patient accounts. The first storage device is part of and/or is operatively coupled to the server device. The second storage device being part of a portable medical device. The portable medical device may be, for example, a glucose meter, an insulin pump, or the like.

According to embodiments, the data synchronization is performed with the help of an application program installed on a patient's mobile device. The application program manages data exchange during synchronization without storing the exchanged data to a storage of the mobile device: only a single copy of the data is stored on the storage of the server device.

According to embodiments the method further comprises disabling, by the account manager, data sharing functions. Data sharing functions are functions which are configured to exchange data between the first account and each of the identified patient accounts. The data belonging to the first account and data belonging to the identified patient accounts are stored on a storage device that is part of and/or is operatively coupled to the server device.

According to embodiments the method further comprises authenticating, by the account manager, the request to deactivate the first account associated with the healthcare provider, and only deactivating the first account in case the request was successfully authenticated. Authenticating the request may imply checking if the request was submitted by the healthcare provider assigned to the first account to be deactivated. The first account is only deactivated if said request was submitted by said healthcare provider. The first account is not deactivated if the request was submitted by another, i.e., unauthenticated person.

According to embodiments the account manager sends a confirmation that the first account and the associated patient accounts are deactivated in response. The confirmation is sent to the instance having submitted the request for deactivating the first account.

According to embodiments, the account manager creates an event log record in a data store. The event log record indicates the deactivation date of each of the identified patient accounts. Said data store may be part of and/or may be operatively coupled to the server device.

According to embodiments the method may further comprise:
- receiving, by the account manager, a request to deactivate a patient account associated with the diabetes care management system;
- providing, by the account manager, a warning notification indicating active sessions of the patient account will be terminated;
- soliciting, by the account manager, a confirmation of the request to deactivate the patient account;
- deactivating the patient account upon receipt of the confirmation of the request to deactivate the patient account;
- sending, by the account manager, an electronic notification to the healthcare provider whose first account is associated with the patient account, where the electronic notification advises the person that the corresponding patient account has been deactivated; and
- creating, by the account manager, an audit record in a data store, where the audit record indicates the deactivation of the patient account.

According to embodiments the method may further comprise the following steps to be executed by the accounts manager:
- receiving, by the account manager, a request to deactivate all portable medical devices of a particular device-type and/or device-type version, the request comprising a device-type-ID and/or a device-type-version;
- identifying all patient accounts having assigned a device-ID of a portable medical device of the type and/or version specified in said request;
- deactivating and unregistering said devices having the type and/or version specified in said request in the identified patient accounts;
- sending an electronic notification to the patients of said identified patient accounts, where the electronic notification advises the person that the corresponding portable medical device has been unregistered and/or needs a firmware update. For example, said notification may be sent to the application programs of said patients for display via a screen of the mobile device.

According to embodiments, at least a portion of the data is encrypted.

According to embodiments, the request to deactivate the patient account originates from the healthcare provider whose first account is associated with said patient account.

According to embodiments the electronic notification is provided to a patient associated with the patient account. The notification may be executed by e-mail, SMS and/or a graphical user interface provided by the account manager and communicated over a network. Said GUI may be displayed by a browser installed on the mobile device of the patient and/or may be displayed by the application program installed on said mobile device.

According to embodiments the method further comprises authenticating, by the account manager, the request to deactivate the patient account prior to deactivating the patient account. The patient account is only deactivated in case the request was successfully authenticated. For example, a request may be successfully authenticated if the account manager receives some data being able to proof that said request was received from said very patient or from a healthcare-provider whose first account is associated to said patient account. Said data may be provided in the form of authentication data, e.g. a password and/or biometrical data.

According to embodiments the patient account is associated with a portable medical device. This may be accomplished, for example, by storing a patient-ID or a patient account-ID in association with a device-ID of said portable medical device. patient data belonging to said patient account is stored on a storage medium that belongs to and/or is operatively coupled to the server device. The method may further comprise disabling, by the account manager, data transfer between the portable medical device and said patient account upon a deactivation of said patient account.

According to embodiments the method further comprises disabling, by the account manager, data transfer between an electronic medical records system and said patient account upon a deactivation of said patient account. As described above, patient data belonging to said patient account is stored on a storage medium that belongs to and/or is operatively coupled to the server device. The method may further comprise providing, by the account manager, an option to cancel the request to deactivate the patient account.

In a further aspect the invention relates to a method for managing accounts in a diabetes management system, comprising:
- receiving, by an account manager, a request to deactivate a patient account associated with the diabetes care management system, where the account manager is implemented as computer executable instructions executing on a computer processor of a server device;
- providing, by the account manager, a warning notification indicating active sessions of the patient account will be terminated;
- soliciting, by the account manager, a confirmation of the request to deactivate the patient account;
- deactivating the patient account upon receipt of the confirmation of the request to deactivate the patient account;
- sending, by the account manager, an electronic notification to a person associated with the patient account, where the electronic notification advises the person that the corresponding patient account has been deactivated; and
- creating, by the account manager, an audit record in a data store, where the audit record indicates the deactivation of the patient account.

The present disclosure provides a method for managing accounts in a diabetes management system. The system can include receiving, by an account manager, a request to deactivate a first account associated with a healthcare provider. The first account may be associated with a plurality of patient accounts. The account manager may be implemented as computer executable instructions executing on a computer processor of a server device. In various aspects, the method can include removing, by the account manager, access to data associated with the first account. Each of the patient accounts associated with the first account are identified by the account manager and deactivated. An electronic notification may be sent by the account manager to a person associated with each of the identified patient accounts. The electronic notification may advise the person that the corresponding patient account has been deactivated. The method may include creating, by the account manager, an audit record in a data store, where the audit record indicates the deactivation of the first account.

In various other aspects, the method for managing accounts in a diabetes management system comprises receiving, by an account manager, a request to deactivate a patient account associated with the diabetes care management system. The account manager may be implemented as computer executable instructions executing on a computer processor of a server device. The account manager may provide a warning notification indicating active sessions of the patient account will be terminated. The method can include soliciting, by the account manager, a confirmation of the request to deactivate the patient account. The patient account may be deactivated upon receipt of the confirmation of the request to deactivate the patient account. The account manager may send an electronic notification to a person associated with the patient account, where the electronic notification advises the person that the corresponding patient account has been deactivated. The method can include creating, by the account manager, an audit record in a data store, where the audit record indicates the deactivation of the patient account.

According to embodiments the above described method may be computer-implemented.

Further areas of applicability will become apparent from the description provided herein. The description and specific examples in this summary are intended for purposes of illustration only and are not intended to limit the scope of the present disclosure.

In a further aspect, not being part of the claimed invention, the disclosure relates to a security system for medical data comprising:
- a portable medical device for measuring medical parameters being indicative of a physiological state of a mammal; and
- a server device, the server device comprising and/or being operatively coupled to a storage device, the server device hosting an accounts manager of a diabetes management system, wherein the accounts manager is configured to manage accounts stored on said storage device, said accounts comprising a first account associated with a healthcare provider and a plurality of patient accounts associated with said first account, each patient account comprising medical data of a respective patient;
- a mobile device hosting an application program being interoperable with the portable medical device and with the accounts manager, the mobile device being connected with the server device via a network. The portable medical device is configured to repeatedly measure medical parameters of one of said patients.

The application program is configured to repeatedly and fully automatically receive the measured medical parameters from said measurement device and is configured to immediately forward the received measured medical parameters to the accounts manager for storing said parameters on the storage device of the server as part of the patient account of said patient,

The portable medical device, the application program and the accounts manager are configured such that only a single copy of the measured medical data is permanently stored in said storage device and such that any temporally created copies of said measured medical data are immediately erased from the portable medical device and the mobile device after execution of the forwarding.

The account manager is configured to execute the following steps:
- receiving a request to deactivate a first account;
- removing access to data associated with the first account;
- identifying each of the patient accounts associated with the first account and deactivating each of the identified patient accounts;
- sending an electronic notification to the patients associated with each of the identified patient accounts, where the electronic notification advises the patient that the corresponding patient account has been deactivated;
- creating an audit record in a data store, where the audit record indicates the deactivation of the first account; and
- sending a termination command to the application programs of the patients associated with each of the identified patient accounts, wherein the application programs are configured such that they immediately terminate forwarding of medical measurement data to the accounts manager upon receiving the termination command.

According to embodiments, the portable medical device comprises a processor and an interface for exchanging data with the application program. The interface is capable of pairing the portable medical device to the application program. In a paired state the portable medical device and the application program are connected via a data communication connection. Only in the paired state the one portable medical device is configured for enabling a transferring of measurement data to the application program.

According to embodiments, the application program is configured to execute steps comprising:
- receiving a pairing command from the patient via a user interface or from the portable medical device;
- in response to the receiving of the pairing command, requesting a device-ID from the portable medical device;
- in response to said request, receiving the device-ID from the portable medical device;
- evaluating the received device-ID for determining if a pairing between the application program and the medical measuring device has already been established at least once previously;
- Only if it is determined that the pairing between the application program and the medical measuring device has already been established at least once previously, automatically establishing the pairing in interoperation with the portable medical device by creating the data communication connection; and automatically executing the forwarding of the measurement data;
- Only if it is determined that the pairing between the application program and the medical measuring device has not yet been established at least once previously, automatically sending a device-registration request to the accounts manager, the device registration request comprising the received device-ID and a patient-ID of; and only executing the forwarding of the measurement data after having received a confirmation from the accounts manager that the portable medical device was registered for said patient at the accounts manager successfully.

According to embodiments, the application program is configured such that in response to receiving the confirmation from the accounts manager that the portable medical device was successfully registered for said patient at the accounts manager, permanently storing said device-ID in a storage medium of the mobile device as a reference value in association with the confirmation of the accounts manager; and in response to receiving a later pairing command, determining, by comparing the stored reference device-ID with a device-ID received from the medical measuring device, that a pairing between the application program and the medical measuring device has already been established.

According to embodiments, the application program is configured such that receiving the device-ID from the portable medical device comprises:
- receiving a message from the portable medical device that no device-ID can be returned to the application program;
- automatically creating a random character string;
- returning the created random character string to the portable medical device for permanent storage in a storage of said portable medical device as a device-ID of said measurement device;
- using the created random character string as the received device-ID.

According to embodiments, the accounts manager is configured such that successfully registering the portable medical device at the accounts manager comprises:
- Authenticating the patient;
- Receiving a confirmation from the authenticated patient that the portable medical device identified by the device-ID in the device registration request shall be registered;
- if said confirmation is received, storing the device-ID contained in the device-registration request by the accounts manager in association with the patient-ID in the device registration request, the patient-ID identifying the patient account of said patient.

According to embodiments, the accounts manager is configured such that only measurement data collected by a portable medical device having successfully been registered at the accounts manager is received and stored in association with a respective patient account. In addition, or alternatively, the application program is configured such that only measurement data collected by a portable medical device having successfully been registered at the accounts manager is forwarded to the accounts manager.

According to embodiments, the medical measuring device is a glucose meter.

According to embodiments, the account manager is configured to execute, upon receiving the request to deactivate the first account, the steps of:
- disabling data synchronization functions, the data synchronization functions being configured to exchange data between a first and a second storage device, the exchanged data belonging to the first account and/or one of the identified patient accounts, the first storage device being part of and/or being operatively coupled to the server device, the second storage device being part of the medical measuring device; and/or
- disabling data sharing functions, the data sharing functions being configured to exchange data between the first account and each of the identified patient accounts, wherein the data belonging to the first account and to the identified patient accounts are stored on a storage device that is part of and/or is operatively coupled to the server device.

### DRAWINGS

The drawings described herein are for illustrative purposes only of selected embodiments and not all possible implementations, and are not intended to limit the scope of the present disclosure.
Figure 1 is a diagram depicting an exemplary diabetes management system;
Figure 2 is a diagram depicting a software architecture system for the diabetes management system;
Figure 3 is a diagram depicting an alternative embodiment of the diabetes management system;
Figure 4 is a diagram depicting an alternative embodiment of the software architecture system for the diabetes management system shown in Figure 3;
Figure 5 is a schematic diagram illustrating various operations that may be performed in connection with the closing of a healthcare provider account associated with the diabetes management system;
Figure 6 is a schematic diagram illustrating various operations that may be performed in connection with the closing of a patient account associated with a healthcare provider and the diabetes management system; and
Figures 7 - 11 illustrate various graphical user interfaces that may be used in
connection with the diabetes management system, portal, and software architecture.

Corresponding reference numerals indicate corresponding parts throughout the several views of the drawings.

### DETAILED DESCRIPTION

The following embodiments merely define examples, only those falling within the scope of the claims are part of the invention.
Example embodiments will now be described more fully with reference to the accompanying drawings.

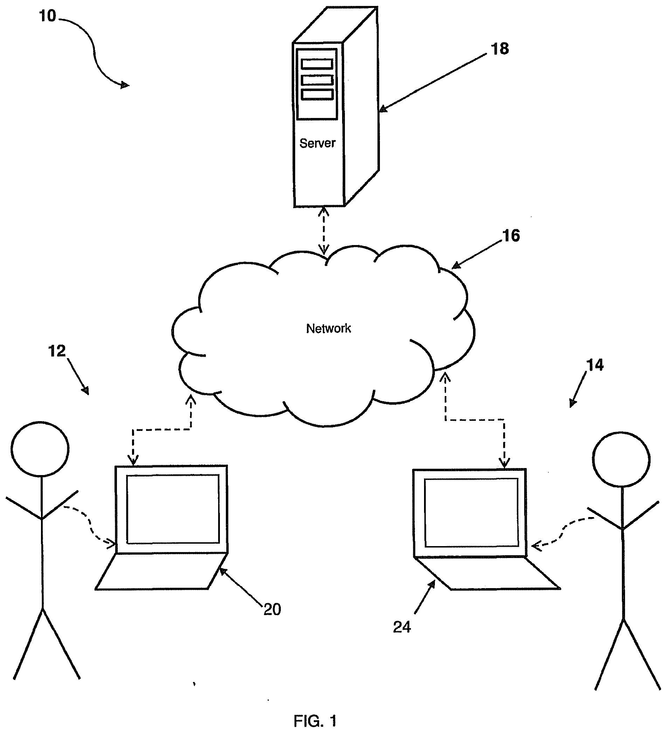

Figure 1 illustrates an exemplary diabetes management system ("DMS") 10 for storing and transmitting medical data and information across a distributed computing environment. By way of non-limiting example, such data and information might include blood glucose measurements, search engine functionality, alerts and reminders, user-entered notes, reports and graphs, and global positioning system information.

The diabetes management system 10 may include generally a patient data system 12, a healthcare professional data system 14, a network 16, and at least one server computer 18. The terms 'server computer', 'server' and 'server device' are used herein synonymously. As will be explained in more detail below, the diabetes management system 10 may be configured such that data and information is electronically sent and received to and from the patient data system 12, the healthcare professional data system 14, and the server computer 18 via the network 16.

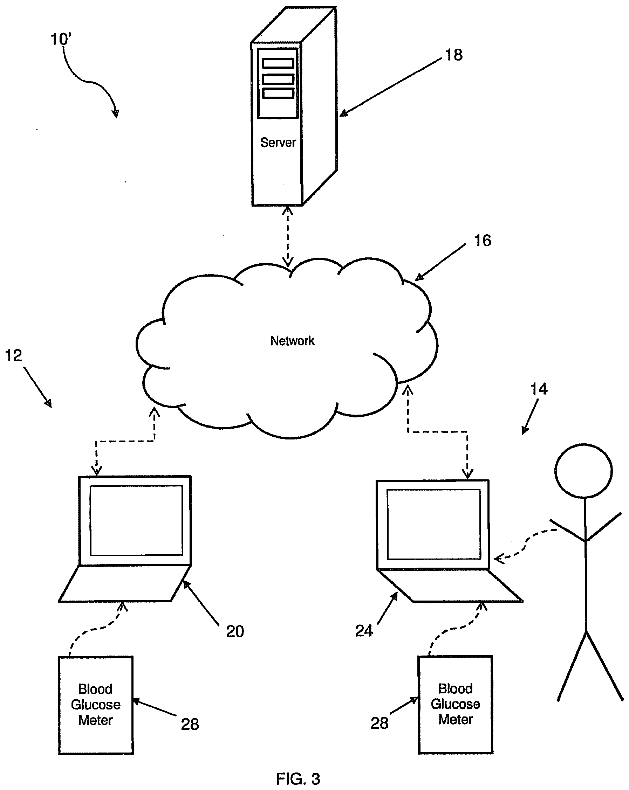

The diabetes management system 10' illustrated in Figure 3 may also generally include a medical data recording device 28, such as a blood glucose meter, for measuring and storing certain medical data or information, such as blood glucose measurements. The 'data recording device ' is also referred herein as 'medical device'. Preferentially, the medical device is a portable medical device. The medical data recording device 28 may be operable to send medical data or information to at least one of the patient computing device 20 and the healthcare professional computing device 24 through a data transmission device, such as a hard-wired data port or a wireless data port such as a Bluetooth receiver, incorporated therein. The medical data recording device 28 may be associated with a unique identifier or security code that attaches to any data transmitted by the medical data recording device. Accordingly, in this method of data transmission, the patient or other user may not be required to enter a username, password, or other security code prior to transmitting data via the network 16.

The patient data system 12 may include at least one patient computing device 20 operably connected to, and in communication with, the network 16 through a wired or wireless connection such as WiFi. The computing device 20 is also referred herein as mobile device. By way of example, the patient computing device 20 may be a mobile device such as an electronic tablet or a smartphone. The patient computing device 20 may include a data input device, a processor, a memory, and an output device. The data input device may be a touchscreen, a keyboard, a mouse, a microphone, a hard-wired data port such as a universal serial bus port, or a wireless data port such as a Bluetooth receiver. The processor may be connected to the data input device, the memory, and the output device. In an example embodiment, the processor includes a general purpose processor. In another example embodiment, the processor includes an application specific integrated circuit. The output device includes a display, a speaker, or the like.

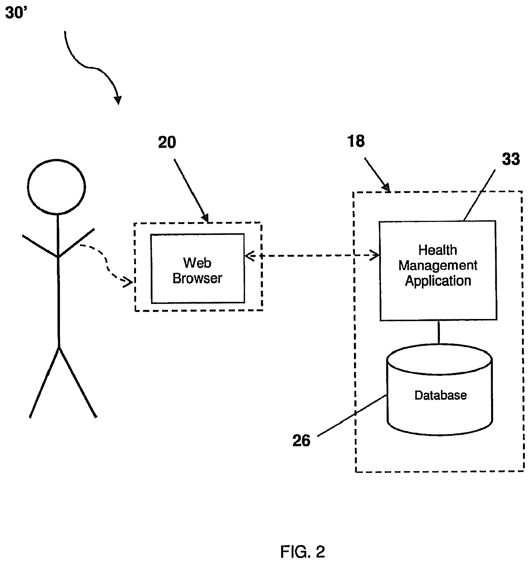

With reference to Figures 1 and 2, in one embodiment of the patient data system 12, a user (e.g., a patient) may enter data or information such as blood glucose measurements into a web browser on the patient computing device 20 via the data input device for transmittal via the network 16. Prior to entering or transmitting data via the network 16, the user may be required to enter a username, password, or other security code, in order to ensure the accurate transmission and storage of data, as will be described in more detail below.

The healthcare professional data system 14 may include at least one healthcare professional computing device 24 operably connected to, and in communication with, the network 16 through a wired or wireless connection, such as WiFi. A physician or other person with access to the healthcare professional computing device 24 may utilize the network 16 to download and view data or information such as blood glucose measurements that are stored on the server computer 18. By way of example, the healthcare professional computing device 24 may be a desktop computer or a mobile communication device such as an electronic tablet or a smartphone. The healthcare professional computing device 24 may include a data input device, a processor, a memory, and an output device. The data input device may be a touchscreen, a keyboard, a mouse, a microphone, a hard-wired data port such as a universal serial bus port, or a wireless data port such as a Bluetooth receiver. The processor may be connected to the data input device, the memory, and the output device. In an example embodiment, the processor includes a general purpose processor. In another example embodiment, the processor includes an application specific integrated circuit. The output device includes a display, a speaker, or the like.

With reference to Figures 1 and 2, the server computer 18 may include a processor, an input device, an output device, and a memory including a database 26. The processor is connected to the memory, the input device, and the output device. In an example embodiment, the processor includes a general purpose processor. In another example embodiment, the processor includes an application specific integrated circuit. The input device includes a keyboard, a mouse, a touchpad, a trackpad, or the like. The output device includes a display, a speaker, or the like. The server computer 18 and database 26 may be operably connected to, and in communication with, the network 16 through a wired or wireless connection. The server computer 18 may be operable to send and receive via the network 16 data, such as blood glucose measurements received from the patient data system 12, to the database 26 for storage and later retrieval. As will be discussed below, data and information sent via the network 16 may be assigned to a unique patient account prior to being stored in the database 26 located on the server computer 18.

Figure 3 illustrates an alternative embodiment of the diabetes management system 10'. The alternative embodiment of the diabetes management system 10' shown in Figure 3 may be similar to the embodiment shown in Figure 1, and include a patient data system 12, a healthcare professional data system 14, a network 16, and at least one server computer 18. The patient data system 12 may include at least one patient computing device 20 operably connected to, and in communication with, the network 16 through a wired or wireless connection such as WiFi. The healthcare professional data system 14 may include at least one healthcare professional computing device 24 operably connected to, and in communication with, the network 16 through a wired or wireless connection, such as WiFi, through which a physician or other person with access to the healthcare professional computing device may download and view data or information such as blood glucose measurements sent from the patient computing device 20 via the network 16.

The medical data recording device 28 may be configured to transmit data to at least one of the patient computing device 20, the healthcare professional computing device 24, and the server computer 18 at regular, programmable intervals, at varying times chosen by the user, in real time as blood glucose measurements are taken, or when blood glucose measurements reach certain threshold levels that may be set by the patient or the healthcare professional.

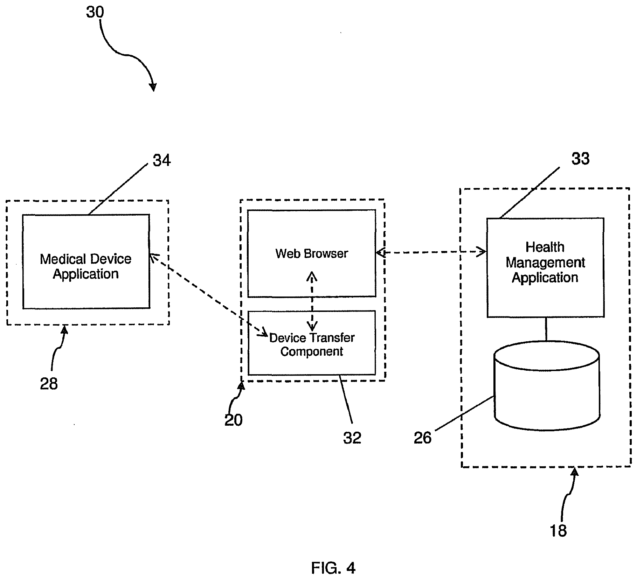

With reference to Figure 4, one embodiment of a software architecture system 30 for use with the diabetes management system 10 is illustrated. The software architecture system 30 may be operable to allow transmission and manipulation of data and information, such as blood glucose measurements, from the medical data recording device 28, through the network 16 and between the patient data system 12, the server computer 18, and the healthcare professional data system 14.

The software architecture system 30 may include a 'device transfer component' 32 that is also referred herein as 'application program' hosted by and running on the mobile device 20. The device transfer component 32 is a piece of program logic stored on the server computer 18 for transmission through the network 16 and installation on the patient computing device 20 and the healthcare professional computing device 24. The device transfer component may be provided for download e.g. in the form of an 'app'. Installation of the device transfer component 32 onto the patient computing device 20 or the healthcare professional computing device 24 will allow such device to send data and information, such as blood glucose measurements, to the health management application 33 installed on the server computer 18. When the medical data recording device 28 sends data to the patient computing device 20 or the healthcare professional computing device 24, the device transfer component 32 is operable to pass the data to the server computer 18 via the network 16, without storing the data in the device transfer component 32 or the patient computing device 20. A medical device application 34 may be installed on the medical data recording device 28, also referred herein as 'portable medical device', to facilitate the exchange of data and information with the device transfer component 32, as described above. Data sent via the device transfer component 32 may include an identifier code, a device-ID, that is unique to the particular medical data recording device 28 from which it was received. The medical data recording device may uniquely be assigned to a patient and in this case the device-ID may act as a patient-ID, e.g. for identifying the patient account to which the measurement data gathered by the data recording device shall be transferred and stored. The device-ID acting as patient-ID may allow the device transfer component 32 and the database 26 to efficiently send, store, and retrieve data from unique locations, or patient accounts, within the database 26.

Prior to transferring data and information through the network 16, a user may be required to link or associate the medical data recording device 28 with a unique patient account created on the server 18. Authentication may be accomplished by first allowing the medical data recording device 28 to communicate with a patient computing device 20 via an input device such as a USB port or a Bluetooth receiver. The medical data recording device 28 may be assigned a unique identification code or authentication token. Said token may be an XML file, e.g. a SAML token, or the like. After downloading and running the device transfer component 32, and allowing the medical data recording device 28 to communicate with the personal computing device 20, the medical data recording device's authentication token can be assigned to the appropriate patient account. Once the medical data recording device's 28 authentication token has been assigned to the patient account, data and information can be transferred through the device transfer component 32 and assigned to the appropriate patient account in the database 26.

In an alternative embodiment of the data transfer process, the device transfer component 32 may be assigned a unique identification code. Prior to transferring data and information from a medical data recording device 28 through the network 16, the user may be required to associate the device transfer component's 32 unique identification code with the patient's account. Data and information from the medical data recording device 28 can be linked to the device transfer component's 32 unique identification code prior to transmission through the network, and thus assigned to the appropriate patient account in the database 26.

After data has been transferred from the device transfer component 32 to the patient account located on the server 18, the software architecture system 30 may allow such data to be further transferred from the patient account to authorized health professional computing devices 24 and patient computing devices 20 via the device transfer component 32. Transfer of data from the patient account located in the database 26 on the server 18 may require linking the patient account with the device transfer component 32 installed on the health professional computing device 24. The software architecture system 30 may also allow a patient, healthcare professional, or other user to create customized reports related to the data and information contained therein or perform research with respect to information contained within or outside of the software architecture system. In addition, the software architecture system 30 may be operable to provide data backup and restoration services with respect to data and information that may have been lost from the patient data system 12 or the healthcare professional data system 14.

With reference to Figure 2, in another embodiment of the software architecture system 30', the user may send and receive data from the server computer 18 and the database 26 via a web browser on the patient computing device 20, in lieu of downloading and utilizing the device transfer component 32 on the patient computing device. In the software architecture system 30', the server computer 18 may include the device transfer component 32, allowing the user to send data and information to the server computer 18 for storage in the database 26, and for further transmission to the healthcare professional computing device 24 via the network 16. The user may access the diabetes management system 10 and the software architecture system 30' by entering a URL designated for the diabetes management system directly into a web browser.

Prior to transferring data through the network 16 to the server computer 18, the user may be required to create a unique patient account and security credentials, such as a username and password, to ensure a unique and secured storage location in the database 26. The user may also be required to associate, or link, the medical data recording device 28 authentication token with the patient account. Once the user has created the patient account and linked the medical data recording device 28 authentication code to the patient account, the user may be permitted to transfer data and information to and from the server computer 18 without otherwise logging into the patient account. In one aspect of the diabetes management system 10, access to the server 18 via the web browser may allow a user to transmit data such as blood glucose measurements, patient weight, meal information, and similar information, to the patient account in the database 26. In another aspect of the diabetes management system 10, access to the server 18 via the web browser may allow the user to create and view reports, graphs, and other information based at least in part on data transmitted from the user.

Communication between the patient data system 12, the healthcare professional data system 14, and the server computer 18 may utilize HTTP basic authentication in combination with secure sockets layer (SSL) security protocol. Other communication and security protocols known in the art are also contemplated.

Operation of the diabetes management system 10 may include a user operating a web browser to access the server computer 18 via the network 16. For example, a user may create a unique patient account on the server computer 18 utilizing security credentials such as a username and password. Creation of a patient account may allow the user to send and store data and information in a secure location within the database 26 on the server computer 18.

In other aspects, a user may operate a web browser to access the server computer 18 in order to download the device transfer component 32 onto at least one patient computing device 20 and/or at least one health professional computing device 24. A user may register the medical data recording device 28 with at least one of the device transfer component 32 and the patient account. Registration of the medical data recording device 28 with the device transfer component 32 and the patient account may be accomplished by allowing the medical data recording device 28 to communicate with a patient computing device 20 via an input device such as a USB port, a Bluetooth receiver, or other wired or wireless technology. Each medical data recording device 28 may be assigned a unique identification code or authentication token that can be associated with the patient account, thus ensuring that data and information sent from the medical data recording device 28 is stored only in the patient account. It is also contemplated that registration of the medical data recording device 28 with the device transfer component 32 and the patient account may be accomplished by the user directly entering the medical data recording device's identification code or authentication token directly into device transfer component 32 or a web browser.

In various aspects, a user may send data and information from the medical data recording device 28 to the device transfer component 32. Data and information may be sent via an input device such as a USB port or a Bluetooth receiver on the patient computing device 20 or the health professional computing device 24. Data and information received from the medical data recording device 28 may include the identification code or authentication token assigned to the particular medical data recording device. In this way, the diabetes management system 10 can ensure the integrity of data and information sent to the patient account.

The device transfer component 32 may send data and information received from the medical data recording device 28 to the server 18. In certain aspects, it is contemplated that the device transfer component 32 may not necessarily store or otherwise record in memory the data and information received from the medical data recording device 28. Rather, the device transfer component 32 may function as an intermediary between the medical data recording device 28 and the server 18, facilitating the transfer of data and information therebetween. Data and information transferred from device transfer component 32 may be authenticated and directed to the patient account through the associated identification code or authentication token received from the medical data recording device 28, as explained above. Data and information associated with the patient account in the server computer 18 database 26 may be transferred to at least one of the patient computing device 20 and the health professional computing device 24 via the network 16. The transfer of data from the server computer 18 may first require authentication.

In various aspects, the present technology provides a web-portal by which the owners of the diabetes management system perform various functions related to healthcare provider accounts, patient accounts, or both. In other aspects, a web browser or web-based application may be used to perform similar functions. For example, a healthcare provider may be set up with a first, or main account to help manage treatment of a plurality of different patients, each of which has a respective patient account accessible from the server computer 18 associated with the diabetes management system. The healthcare provider may be able to configure its account to display certain data, statistics, and/or usage data from the various patients having patient accounts accessible through the healthcare provider's account. A review of the data may allow the healthcare provider to better assess the patients' health, and adjust treatment regimens based on the recent and historical data.

By utilizing the diabetes management system 10, healthcare providers and patients may readily store, share, and later access medical information relating to the patients, for example, to analyze historical information regarding a subject's biological condition, operation of the patient's portable medical device 28, treatment, treatment results, personal habits, or the like. Based on such historical data, the healthcare provider and/or patient may be able to recognize trends, beneficial practices, detrimental practices or the like and, thereby, adjust or design treatment plans that take advantage of beneficial trends and practices and avoids detrimental trends and practices.

The diabetes management system 10 may include software for generating or otherwise providing reports containing information received from a patient, a group of patients, or multiple groups of patients within the same healthcare provider account. In this manner, a patient or a patient's healthcare provider may readily access formatted reports of information regarding the patient's condition, historical condition, the patient portable medical device operation or condition, or the like, or similar information regarding one or more defined groups of patients. Reports may be formatted in various pre-defined formats provided by the diabetes management system 10. Alternatively or in addition, the system 10 may allow patients and/or healthcare providers to design their own report format, including determining what type of information to include in the report and how the information is filtered, presented, displayed, etc. Various aspects of the present disclosure are directed a comprehensive system capable of collecting and managing patient information for multiple patients, the multiple patients with a plurality of different types of medical devices.

The sharing of accounts and data, as well as the maintaining of a suitable data repository can be managed by various administrative guidelines. Such administrative guidelines may need to follow certain procedures for opening, closing, and deactivating both patient and healthcare provider accounts associated with the data sharing and storage.

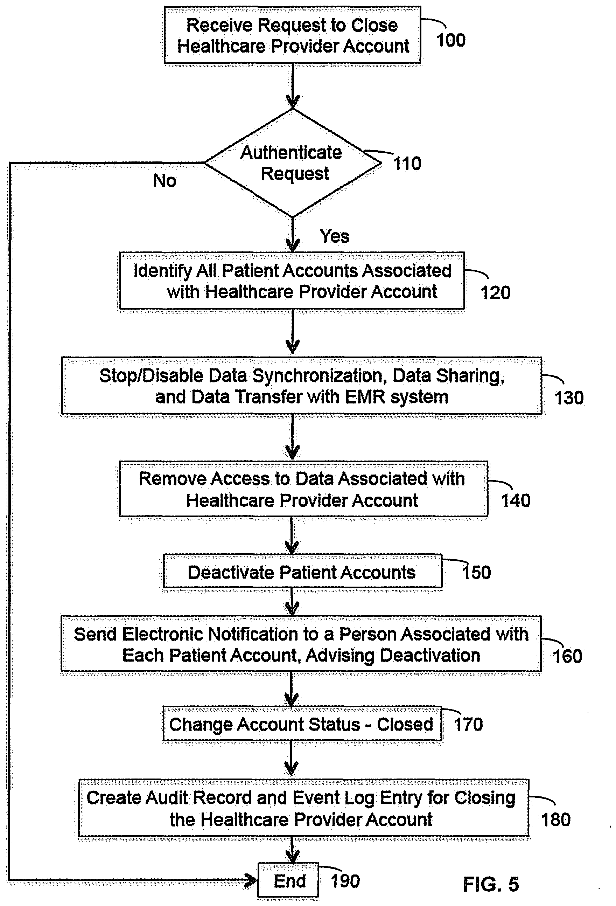

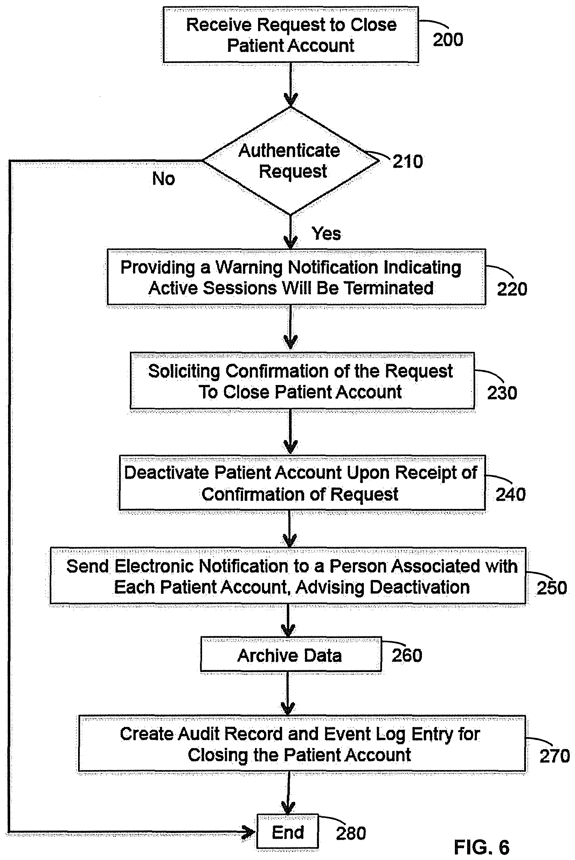

With general reference to Figures 5 and 6, the web-portal or web-based browser/applications by which either the owners of the diabetes management system or the healthcare providers themselves perform various functions related to healthcare provider accounts, patient accounts, or both can be provided with certain predetermined procedures to activate and deactivate accounts, as well as link healthcare provider accounts with a plurality of patient accounts. It should be understood that Figures 5 and 6 are merely exemplary in nature, and are not mutually exclusive aspects. For example, certain features shown with the method represented in Figure 5 may also be performed with the method represented in Figure 6, and vice versa, even though they are not specifically shown. Further, not each feature is necessarily a required step of the respective method, and the order in which the method steps are performed should not be limited to the order specifically depicted in the figures or discussed herein.

Figure 5 generally represents one non-limiting process associated with the closure or deactivation of a healthcare provider account. As represented by method box 100, an account manager can receive a request to deactivate a first account associated with a healthcare provider, or a healthcare provider account, which in turn may be associated with a plurality of patient accounts that may be linked to the healthcare provider account. As used herein, the term "account manager" refers to the implementation of computer executable instructions executed on a computer processor of a server device, or the like. By way of example, an account manager may include a web-based portal or a web-based application, and may include various functionality to perform logic-based operations.

At various points during the deactivation or account closing process, the request may be authenticated, as represented by method box 110. Authentication may be accomplished using various techniques, including, but not limited to authentication of username and/or password credentials, permissions, unique identifiers, security questions, or other identifications. Authentication can also include various requests to one or more users seeking a confirmation or acknowledgement of the proposed request.

The healthcare provider account may generally be associated with a plurality of patient accounts that are linked by the healthcare provider or diabetes management system operator. For example, patient accounts may be provided with a unique identifier, such as a number, code, or token. At some point, the healthcare provider account would be linked together with various patient accounts by association of the healthcare provider account with the unique identifier for each patient account. Additionally or alternatively, the healthcare provider accounts may have the unique identifier. In certain aspects, associating patient accounts with the healthcare provider account may be performed by the healthcare provider using the healthcare provider computing device, or may be performed by a diabetes management system operator using a web-portal. The information associating healthcare provider accounts with patient accounts may be stored in an appropriate data store.

Once the request for deactivation or closure of a healthcare provider account is received and/or authenticated, the present technology may identify all of the patient accounts associated with the healthcare provider account as referenced by method box 120. For example, the patient accounts may be filtered out based on the associated healthcare provider account. In addition to being filtered by the healthcare provider, patient accounts can additionally be filtered by diabetes type, geographic location, age, gender, therapy type, user type, etc. As referenced by method box 130, various data synchronization activities, data sharing, and data transfers between accounts may be interrupted, stopped, and/or disabled, by the account manager, between the healthcare provider account and the diabetes management system, as well as each of the identified patient accounts. Data synchronization activities may include, but are not limited to, processes of establishing consistency among data between patient accounts and the healthcare provider account, and the continuous harmonization of the data over time. These activities may include synchronizing files; data; updates; settings; preferences; formats; statistics; usage information; device information; configurations; messages; notes; etc. In further aspects, data sharing functions may also be stopped and disabled, by the account manager, between the healthcare provider account and the identified patient accounts. Data sharing functions may include, but are not limited to, providing read and/or write functions and access to medical, usage, and personal data; bibliographic information; statistics; etc. between the healthcare provider account and the identified patient accounts and/or patient medical devices.

To the extent any data transfer activities took place between the healthcare provider account and/or patient accounts, those transfer activities may be disabled by the account manager after a request to close or deactivate an account. For example, certain healthcare provider accounts and/or patient accounts may be associated, linked, or otherwise work with electronic medical records (EMR) systems as commonly known in the art. In the event a healthcare provider account or patient account is closed or deactivated, appropriate notifications may need to be provided to EMR systems or other user interfaces as may be required to stop pending or future data transfers between the EMR systems and healthcare provider accounts and/or patient accounts, or to indicate that such accounts are no longer available for access by the EMR system.

As indicated by method boxes 140 and 150, upon receiving a request to deactivate or close a healthcare provider account, the account manager may deny or remove further access to the data associated with the healthcare provider account, and deactivate certain or all of the patient accounts associated with the healthcare provider account.

In certain aspects, the methods for managing accounts includes sending, by the account manager, an electronic notification to a person associated with each of the identified patient accounts, such as the patient, where the electronic notification advises the person that the corresponding patient account has been deactivated, and to inform them that the healthcare provider office has stopped data sharing with them. By way of example, and with reference to method box 160, the electronic notification may be via electronic mail or other electronic messaging service. As indicated by method box 170, the status of the healthcare provider account may be changed to a "closed" status, and the closure date may be recorded. Messages may be displayed to one or more users informing them that the healthcare provider account is closed and all associated patient and user accounts are deactivated. Various audit records and event log entries may be created by the account manager that can be saved and/or archived in a data store within the system, as shown by method box 180, to indicate and memorialize various information related to the closed healthcare provider and/or patient account(s). Once closed or deactivated, the web-based portal or web-based application may display a message to inform a user that various accounts are deactivated and that the user details and other identifying features can no longer be edited. It should be understood that various opportunities may be provided throughout this process enabling a user to cancel one or more operations associated with the closing of the accounts.

In additional to typical logistical and testing information generally regarded as useful for diabetes treatment and management control, the web-portal may be configured to collect and use geographic locations of the healthcare provider and/or the patients in order to comply with certain regulatory and/or compliance regimes that may exist for different geographic locations, such as different states or countries. By way of example, different geographic locations may have different requirements concerning the archiving of data related to medical treatment. In various aspects, the computer-implemented methods of the present technology can be configured to archive data associated with each of the plurality of patient accounts and healthcare provider accounts for at least a predetermined period of time. The predetermined period of time may be specifically affiliated with regulations or compliance standards for the geographic location, which may be recorded by the system for each account and managed by the owner of the diabetes management system. Various portions of the data such as references to medical patient data and personal data may be encrypted for patient security and confidentiality.

Figure 6 generally represents one non-limiting process associated with the closure or deactivation of a patient account, where the user initiating the closure may be authenticated as a healthcare provider. As represented by method box 200, an account manager can receive a request to deactivate or close a specific patient account associated with a healthcare provider, or a healthcare provider account. The method may include various confirmations of the deactivation request or authenticate the request as previously described above and noted by method box 210.

With reference to method box 220, when a user selects to deactivate a user account, the computer-implemented system may display a warning message that any active sessions of the deactivated user will be terminated. As shown in method box 230, a specific confirmation may optionally be solicited by the account manager, seeking confirmation of the request to close the patient account. The patient account may then be deactivated and closed upon receipt of the confirmation of the request as depicted in method box 240. Similar to the method of deactivating the healthcare provider account, method box 250 indicates that an electronic notification may be sent by the account manager to a person, such as a patient, associated with the patient account to advise the person that the corresponding patient account has been or will be deactivated and/or closed. Certain data associated with the patient account may be archived as noted in method box 260. The archived data may be stored for a predetermined period of time as discussed above. Appropriate audit records and event log entries documenting the deactivation of the patient account may be created as referenced by method box 270.

In various aspects, the patient account may be associated with a portable medical device, for example, having a unique serial number or identifier associated with the account. Methods according to the present technology may include disabling, by the account manager, data transfer activities associated with the portable medical device affiliated with a closed or deactivated patient account.

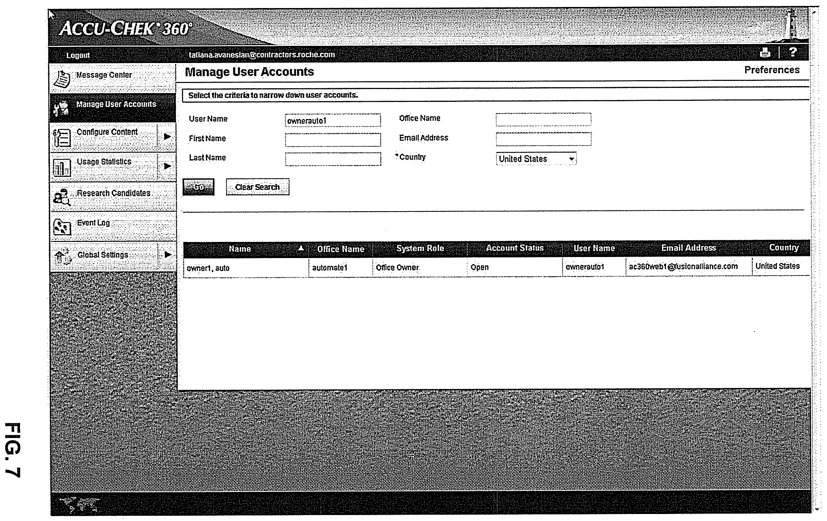

Figures 7 - 11 illustrate various graphical user interfaces that may be used in connection with the diabetes management system, portal, and associated software architecture.

Figure 7 generally depicts management and viewing options for a web-portal user account. Certain criteria can be selected to narrow down the display of user accounts. By way of example, accounts can be sorted by name, office name, system role, account status, user name, associated email address, and country.

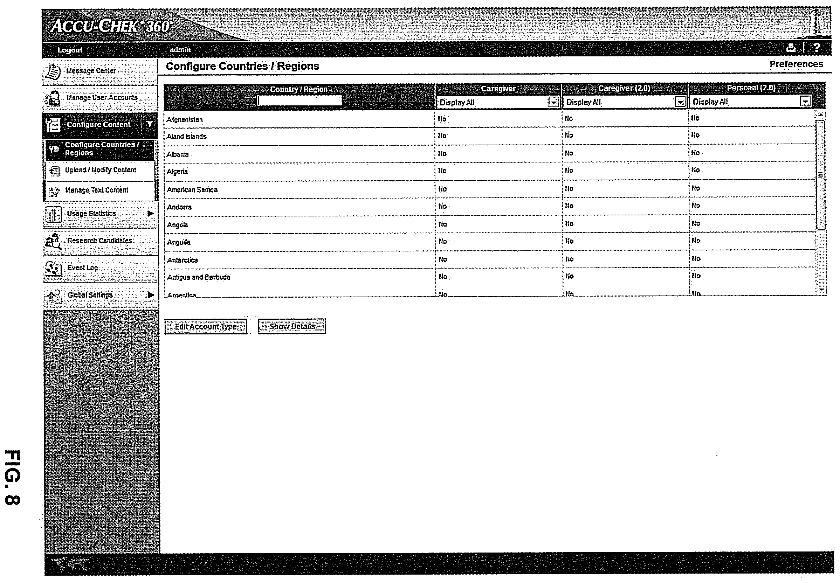

Figure 8 generally depicts the configuration and selection of accounts assigned with countries and/or geographic regions. For example, certain healthcare provider (caregiver) accounts or patient (personal) accounts can be filtered by their association with certain countries and/or geographic regions.

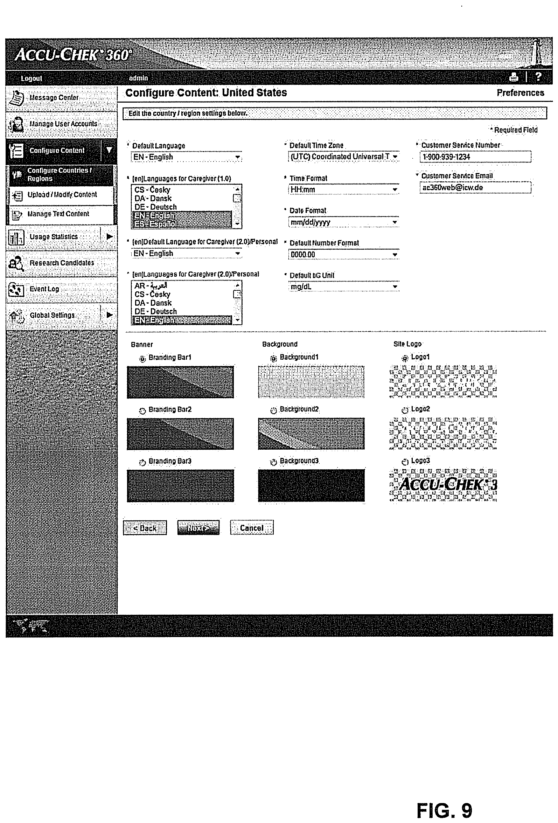

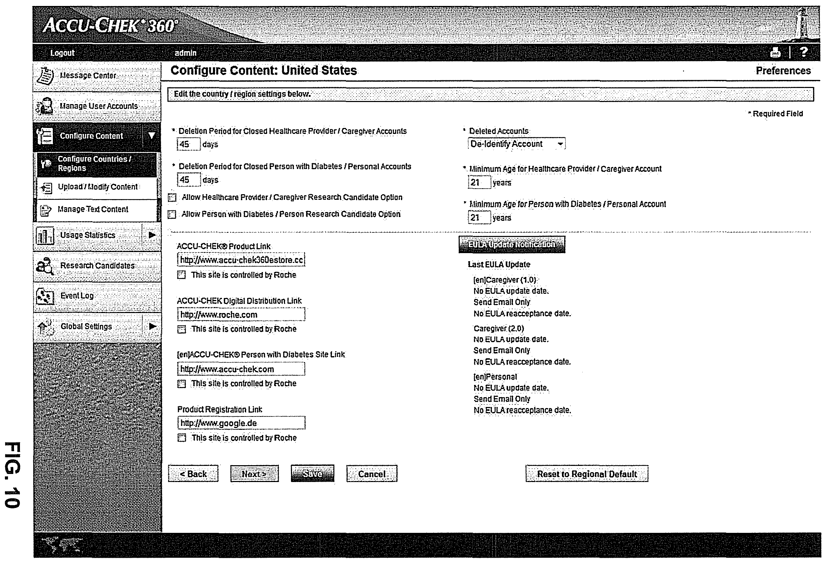

Figures 9 and 10 generally depict additional settings and preferences that may be associated with accounts linked with a specific country or geographic region. As shown, Figure 9 provides a variety of regional settings such as language, time zone and time/date formats, measurement units, logos, color and background selections, and the like. Figure 10 provides configurations for the deletion period for closed accounts, optional selection of research candidates, notations regarding minimum age requirements for account holders, and the like. Figure 10 also provides optional information regarding medical device/product information, registration information, and End User License Agreements.

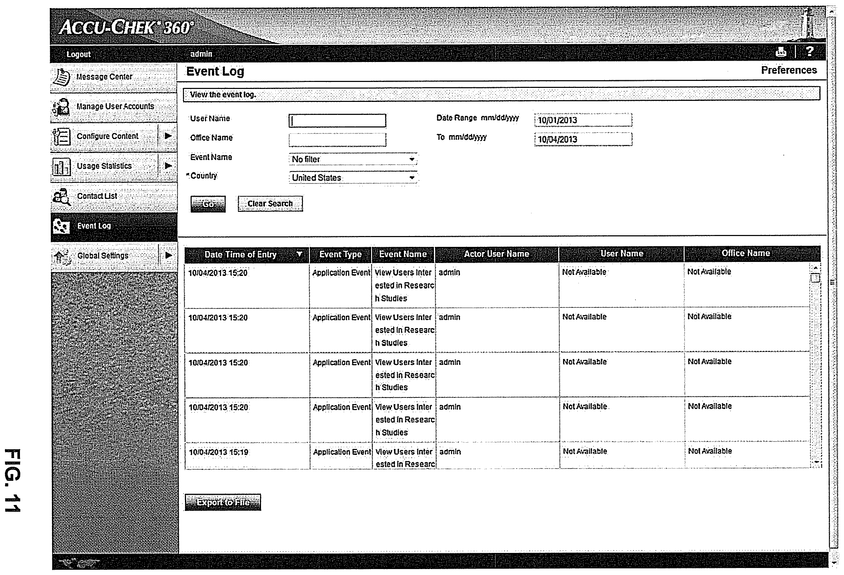

Figure 11 provides an exemplary user interface for an event log. As shown, each event log record can include various bibliographic information, as well as description fields that display event types, names, details, user names and office names/locations.

The techniques described herein may be implemented by one or more computer programs executed by one or more processors. The computer programs include processor-executable instructions that are stored on a non-transitory tangible computer readable medium. The computer programs may also include stored data. Non-limiting examples of the non-transitory tangible computer readable medium are nonvolatile memory, magnetic storage, and optical storage.

Some portions of the above description present the techniques described herein in terms of algorithms and symbolic representations of operations on information.

These algorithmic descriptions and representations are the means used by those skilled in the data processing arts to most effectively convey the substance of their work to others skilled in the art. These operations, while described functionally or logically, are understood to be implemented by computer programs. Furthermore, it has also proven convenient at times to refer to these arrangements of operations as modules or by functional names, without loss of generality.

Unless specifically stated otherwise as apparent from the above discussion, it is appreciated that throughout the description, discussions utilizing terms such as "processing" or "computing" or "calculating" or "determining" or "displaying" or the like, refer to the action and processes of a computer system, or similar electronic computing device, that manipulates and transforms data represented as physical (electronic) quantities within the computer system memories or registers or other such information storage, transmission or display devices.

Certain aspects of the described techniques include process steps and instructions described herein in the form of an algorithm. It should be noted that the described process steps and instructions could be embodied in software, firmware or hardware, and when embodied in software, could be downloaded to reside on and be operated from different platforms used by real time network operating systems.

The present disclosure also relates to an apparatus for performing the operations herein. This apparatus may be specially constructed for the required purposes, or it may comprise a general-purpose computer selectively activated or reconfigured by a computer program stored on a computer readable medium that can be accessed by the computer. Such a computer program may be stored in a tangible computer readable storage medium, such as, but is not limited to, any type of disk including floppy disks, optical disks, CD-ROMs, magnetic-optical disks, read-only memories (ROMs), random access memories (RAMs), EPROMs, EEPROMs, magnetic or optical cards, application specific integrated circuits (ASICs), or any type of media suitable for storing electronic instructions, and each coupled to a computer system bus. Furthermore, the computers referred to in the specification may include a single processor or may be architectures employing multiple processor designs for increased computing capability.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

## Claims

1. A computer-implemented method for managing accounts in a diabetes management system (10), comprising:
- receiving, by an account manager (33), a request to deactivate a first account associated with a healthcare provider, where the first account is associated with a plurality of patient accounts and the account manager is implemented as computer executable instructions executing on a computer processor of a server device (18);
- removing, by the account manager, access to data associated with the first account;
- identifying, by the account manager, each of the patient accounts associated with the first account and deactivating each of the identified patient accounts;
- disabling, by the account manager, data synchronization functions, the data synchronization functions being configured to exchange medical measurement data between a first and a second storage device via a network (16), the exchanged data belonging to the first account and/or one of the identified patient accounts, the first storage device being part of and/or being operatively coupled to the server device, the second storage device being part of a portable medical device (28), the portable medical device repeatedly measuring and synchronizing medical parameters of a patient;
- sending, by the account manager, an electronic notification to a person associated with each of the identified patient accounts, where the electronic notification advises the person that the corresponding patient account has been deactivated; and
- creating, by the account manager, an audit record in a data store, where the audit record indicates the deactivation of the first account.

2. The computer-implemented method according to claim 1, further comprising archiving data associated with each of the plurality of patient accounts for a predetermined period of time.

3. The computer-implemented method according to claims 1 or 2 being implemented in a security system for medical data comprising:
- the portable medical device (28) for measuring medical parameters being indicative of a physiological state of a mammal; and
- the server device (18), the server device comprising and/or being operatively coupled to a storage device, the server device hosting an accounts manager of a diabetes management system, wherein the accounts manager is configured to manage accounts stored on said storage device, said accounts comprising a first account associated with a healthcare provider and a plurality of patient accounts associated with said first account, each patient account comprising medical data of a respective patient;
- a mobile device (20) hosting an application program (32) being interoperable with the portable medical device and with the accounts manager, the mobile device being connected with the server device via a network (16);
wherein the portable medical device is configured to repeatedly measure medical parameters of one of said patients;
wherein the application program is configured to repeatedly and fully automatically receive the measured medical parameters from said measurement device and is configured to immediately forward the received measured medical parameters to the accounts manager for storing said parameters on the storage device of the server as part of the patient account of said patient,
whereby the portable medical device, the application program and the accounts manager are configured such that only a single copy of the measured medical data is permanently stored in said storage device and such that any temporally created copies of said measured medical data are immediately erased from the portable medical device and the mobile device after execution of the forwarding;
wherein the account manager is configured to execute the following steps:
receiving a request to deactivate a first account;
removing access to data associated with the first account;
identifying each of the patient accounts associated with the first account and deactivating each of the identified patient accounts;
sending an electronic notification to the patients associated with each of the identified patient accounts, where the electronic notification advises the patient that the corresponding patient account has been deactivated;
creating an audit record in a data store, where the audit record indicates the deactivation of the first account; and
sending a termination command to the application programs of the patients associated with each of the identified patient accounts, wherein the application programs are configured such that they immediately terminate forwarding of the medical measurement data to the accounts manager upon receiving the termination command.

4. The computer-implemented method according to any one of the previous claims 1-3, further comprising disabling, by the account manager, data sharing functions, the data sharing functions being configured to exchange data between the first account and each of the identified patient accounts, wherein the data belonging to the first account and to the identified patient accounts are stored on a storage device that is part of and/or is operatively coupled to the server device.

5. The computer-implemented method according to any one of the previous claims 1-4, further comprising authenticating, by the account manager, the request to deactivate the first account associated with the healthcare provider, and only deactivating the first account in case the request was successfully authenticated.

6. The computer-implemented method of any one of the previous claims 1-5 comprising:
- receiving, by the account manager, a request to deactivate a patient account associated with the diabetes care management system;
- providing, by the account manager, a warning notification indicating active sessions of the patient account will be terminated;
- soliciting, by the account manager, a confirmation of the request to deactivate the patient account;
- deactivating the patient account upon receipt of the confirmation of the request to deactivate the patient account;
- sending, by the account manager, an electronic notification to the healthcare provider whose first account is associated with the patient account, where the electronic notification advises the person that the corresponding patient account has been deactivated; and
- creating, by the account manager, an audit record in a data store, where the audit record indicates the deactivation of the patient account.

7. The computer-implemented method according to any one of claims 1-6, where two or more of the patient accounts are respectively associated with the portable medical device, the method further comprising the following steps to be executed by the account manager:
- receiving a request to deactivate all portable medical devices of a particular device-type and/or device-type version, the request comprising a device-type-ID and/or a device-type-version;
- identifying all patient accounts having assigned a device-ID of a portable medical device of the type and/or version specified in said request;
- deactivating and unregistering said devices having the type and/or version specified in said request in the identified patient accounts;
- sending an electronic notification to the patients of said identified patient accounts, where the electronic notification advises the person that the corresponding portable medical device has been unregistered and/or needs a firmware update.

8. The computer-implemented method according to any one of claims 1-7, where at least a portion of the data is encrypted.

9. The computer-implemented method according to any one of the previous claims 1-8, where the request to deactivate the patient account originates from the healthcare provider whose first account is associated with the patient account.

10. The computer-implemented method according to any one of the previous claims 1-9, where the electronic notification is provided to a patient associated with the patient account.

11. The computer-implemented method according to any one of the previous claims 1-10, further comprising authenticating, by the account manager, the request to deactivate the patient account prior to deactivating the patient account, and only deactivating the patient account in case the request was successfully authenticated.

12. The computer-implemented method according to any one of the previous claims 1-11, where the patient account is associated with a portable medical device, and the method further comprises disabling, by the account manager, data transfer between the portable medical device and said patient account upon a deactivation of said patient account, whereby patient data belonging to said patient account is stored on a storage medium that belongs to and/or is operatively coupled to the server device.

13. The computer-implemented method according to any one of the previous claims 1-12, further comprising:
- disabling, by the account manager, data transfer between an electronic medical records system and said patient account upon a deactivation of said patient account, whereby patient data belonging to said patient account is stored on a storage medium that belongs to and/or is operatively coupled to the server device; and
- providing, by the account manager, an option to cancel the request to deactivate the patient account.

## Patentansprüche

1. Computerimplementiertes Verfahren zum Verwalten von Konten in einem Diabetes-Managementsystem (10), umfassend:
- Empfangen einer Anforderung nach Deaktivierung eines ersten Kontos, das einem Gesundheitsdienstleister zugeordnet ist, durch einen Kontenverwalter (33), wobei das erste Konto einer Mehrzahl von Patientenkonten zugeordnet ist und der Kontenverwalter als von einem Computer ausführbare Anweisungen, die in einem Computerprozessor einer Servervorrichtung (18) ausgeführt werden, implementiert ist;
- Entfernen eines Zugangs zu Daten, die dem ersten Konto zugeordnet sind, durch den Kontenverwalter;
- Identifizieren jedes der Patientenkonten, die dem ersten Konto zugeordnet sind, und Deaktivieren jedes der identifizierten Patientenkonten, jeweils durch den Kontenverwalter;
- Aufheben von Datensynchronisierungsfunktionen durch den Kontenverwalter, wobei die Datensynchronisierungsfunktionen konfiguriert sind, medizinische Messdaten zwischen einer ersten und einer zweiten Speichervorrichtung über ein Netzwerk (16) auszutauschen, wobei die ausgetauschten Daten zu dem ersten Konto und/oder einem der identifizierten Patientenkonten gehören, wobei die erste Speichervorrichtung Teil der Servervorrichtung ist und/oder funktionell mit dieser gekoppelt ist, wobei die zweite Speichervorrichtung Teil einer tragbaren medizinischen Vorrichtung (28) ist, wobei die tragbare medizinische Vorrichtung wiederholt medizinische Parameter eines Patienten misst und synchronisiert;
- Senden einer elektronischen Benachrichtigung an eine Person, die den jeweiligen identifizierten Patientenkonten zugeordnet ist, durch den Kontenverwalter, wobei die elektronische Benachrichtigung der Person mitteilt, dass das entsprechende Patientenkonto deaktiviert wurde; und
- Erzeugen eines Prüfprotokolls in einem Datenspeicher durch den Kontenverwalter, wobei das Prüfprotokoll die Deaktivierung des ersten Kontos angibt.

2. Computerimplementiertes Verfahren nach Anspruch 1, ferner das Archivieren von Daten, die jedem der Mehrzahl von Patientenkonten zugeordnet sind, für eine vorbestimmte Zeitperiode umfassend.

3. Computerimplementiertes Verfahren nach den Ansprüchen 1 oder 2, das in einem Sicherheitssystem für medizinische Daten implementiert wird, das umfasst:
- die tragbare medizinische Vorrichtung (28) zum Messen medizinischer Parameter, die einen physiologischen Zustand eines Säugers angeben; und
- die Servervorrichtung (18), wobei die Servervorrichtung eine Speichervorrichtung umfasst und/oder funktionell mit dieser gekoppelt ist, wobei die Servervorrichtung einen Kontenverwalter eines Diabetes-Managementsystems beherbergt, wobei der Kontenverwalter zum Verwalten von Konten konfiguriert ist, die in der Speichervorrichtung gespeichert sind, wobei die Konten ein erstes Konto, das einem Gesundheitsdienstleister zugeordnet ist, und eine Mehrzahl von Patientenkonten, die dem ersten Konto zugeordnet sind, umfasst, wobei jedes Patientenkonto medizinische Daten eines jeweiligen Patienten umfasst;
- eine mobile Vorrichtung (20), die ein Anwendungsprogramm (32) beherbergt, das zur Zusammenarbeit mit der tragbaren medizinischen Vorrichtung und mit dem Buchhaltungsmanager fähig ist, wobei die mobile Vorrichtung mit der Servervorrichtung über ein Netzwerk (16) verbunden ist;
wobei die tragbare medizinische Vorrichtung zum wiederholten Messen medizinischer Parameter eines der Patienten konfiguriert ist;
wobei das Anwendungsprogramm für ein wiederholtes und voll automatisches Empfangen der gemessenen medizinischen Parameter von der Messvorrichtung konfiguriert ist und zum sofortigen Weiterleiten der empfangenen gemessenen medizinischen Parameter an den Kontenverwalter konfiguriert ist, zum Speichern der Parameter in der Speichervorrichtung des Servers als Teil des Patientenkontos des Patienten,
wobei die tragbare medizinische Vorrichtung, das Anwendungsprogramm und der Kontenverwalter derart konfiguriert sind, dass nur eine einzige Kopie der gemessenen medizinischen Daten auf Dauer in der Speichervorrichtung gespeichert wird, und derart, dass jegliche vorübergehend gebildeten Kopien der gemessenen medizinischen Daten nach der Ausführung des Weiterleitens sofort von der tragbaren medizinischen Vorrichtung und der mobilen Vorrichtung gelöscht werden;
wobei der Kontenverwalter konfiguriert ist, die folgenden Schritte auszuführen:
Empfangen einer Anforderung nach Deaktivierung eines ersten Kontos;
Entfernen eines Zugangs zu Daten, die dem ersten Konto zugeordnet sind;
Identifizieren jedes der Patientenkonten, die dem ersten Konto zugeordnet sind, und Deaktivieren jedes der identifizierten Patientenkonten;
Senden einer elektronischen Benachrichtigung an die Patienten, die den jeweiligen identifizierten Patientenkonten zugeordnet sind, wobei die elektronische Benachrichtigung dem Patienten mitteilt, dass das entsprechende Patientenkonto deaktiviert wurde;
Erzeugen eines Prüfprotokolls in einem Datenspeicher, wobei das Prüfprotokoll die Deaktivierung des ersten Kontos angibt; und
Senden eines Beendigungsbefehls an das Anwendungsprogramm der Patienten, die mit den jeweiligen identifizierten Patientenkonten verbunden sind, wobei die Anwendungsprogramme so konfiguriert sind, dass sie das Weiterleiten der medizinischen Messdaten an den Kontenverwalter sofort beenden, nachdem sie den Beendigungsbefehl empfangen haben.

4. Computerimplementiertes Verfahren nach einem der vorangehenden Ansprüche 1-3, ferner das Aufheben von Datennutzungsverbundfunktionen durch den Kontenverwalter umfassend, wobei die Datennutzungsverbundfunktionen konfiguriert sind, Daten zwischen dem ersten Konto und jedem der identifizierten Patientenkonten auszutauschen, wobei die Daten, die zu dem ersten Konto und zu den identifizierten Patientenkonten gehören, in einer Speichervorrichtung gespeichert werden, die Teil der Servervorrichtung ist und/oder funktionell mit dieser gekoppelt ist.

5. Computerimplementiertes Verfahren nach einem der vorangehenden Ansprüche 1-4, ferner umfassend: Authentifizieren der Anforderung nach Deaktivierung des ersten Kontos, das dem Gesundheitsdienstleister zugeordnet ist, durch den Kontenverwalter und Deaktivieren des ersten Kontos nur in dem Fall, dass die Anforderung erfolgreich authentifiziert worden ist.

6. Computerimplementiertes Verfahren nach einem der vorangehenden Ansprüche 1-5, umfassend:
- Empfangen einer Anforderung nach Deaktivierung eines Patientenkontos, das dem Diabetes-Pflegemanagementsystem zugeordnet ist;
- Bereitstellen einer Warnmeldung, die angibt, dass aktive Sitzungen des Patientenkontos beendet werden, durch den Kontenverwalter;
- Erbitten einer Bestätigung der Anforderung nach Deaktivierung des Patientenkontos durch den Kontenverwalter;
- Deaktivieren des Patientenkontos nach Empfang der Bestätigung der Anforderung zur Deaktivierung des Patientenkontos;
- Senden einer elektronischen Benachrichtigung an den Gesundheitsdienstleister, dessen erstes Konto dem Patientenkonto zugeordnet ist, durch den Kontenverwalter, wobei die elektronische Benachrichtigung der Person mitteilt, dass das entsprechende Patientenkonto deaktiviert worden ist; und
- Erzeugen eines Prüfprotokolls in einem Datenspeicher durch den Kontenverwalter, wobei das Prüfprotokoll die Deaktivierung des Patientenkontos angibt.

7. Computerimplementiertes Verfahren nach einem der Ansprüche 1-6, wobei zwei oder mehr der Patientenkonten jeweils der tragbaren medizinischen Vorrichtung zugeordnet sind, wobei das Verfahren ferner die folgenden Schritte umfasst, die durch den Kontenverwalter auszuführen sind:
- Empfangen einer Anforderung zur Deaktivierung aller tragbaren medizinischen Vorrichtungen eines bestimmten Vorrichtungstyps und/oder einer bestimmten Vorrichtungstypversion, wobei die Anforderung eine Vorrichtungstyp-ID und/oder eine Vorrichtungstypversion umfasst;
- Identifizieren aller Patientenkonten, denen eine Vorrichtungs-ID einer tragbaren medizinischen Vorrichtung des Typs oder der Version wie in der Anforderung angegeben zugewiesen ist;
- Deaktivieren und Abmelden der Vorrichtungen, deren Typ und/oder Version in der Anforderung angegeben ist, in den identifizierten Patientenkonten;
- Senden einer elektronischen Benachrichtigung an die Patienten der identifizierten Patientenkonten, wobei die elektronische Benachrichtigung der Person mitteilt, dass die entsprechende tragbare medizinische Vorrichtung abgemeldet wurde und/oder eine Firmware-Aktualisierung benötigt.

8. Computerimplementiertes Verfahren nach einem der Ansprüche 1-7, wobei zumindest ein Teil der Daten verschlüsselt ist.

9. Computerimplementiertes Verfahren nach einem der vorangehenden Ansprüche 1-8, wobei die Anforderung zur Deaktivierung des Patientenkontos von dem Gesundheitsdienstleister ausgeht, dessen erstes Konto dem Patientenkonto zugeordnet ist.

10. Computerimplementiertes Verfahren nach einem der vorangehenden Ansprüche 1-9, wobei die elektronische Benachrichtigung einem Patienten bereitgestellt wird, der dem Patientenkonto zugeordnet ist.

11. Computerimplementiertes Verfahren nach einem der vorangehenden Ansprüche 1-10, ferner umfassend: Authentifizieren der Anforderung nach Deaktivierung des Patientenkontos durch den Kontenverwalter vor der Deaktivierung des Patientenkontos und Deaktivieren des Patientenkontos nur in dem Fall, dass die Anforderung erfolgreich authentifiziert worden ist.

12. Computerimplementiertes Verfahren nach einem der vorangehenden Ansprüche 1-11, wobei das Patientenkonto einer tragbaren medizinischen Vorrichtung zugeordnet ist und das Verfahren ferner auf eine Deaktivierung des Patientenkontos hin das Aufheben einer Datenübertragung zwischen der tragbaren medizinischen Vorrichtung und dem Patientenkonto durch den Kontenverwalter umfasst, wobei Patientendaten, die zu dem Patientenkonto gehören, in einem Speichermedium gespeichert werden, das zu der Servervorrichtung gehört und/oder funktionell mit dieser gekoppelt ist.

13. Computerimplementiertes Verfahren nach einem der vorangehenden Ansprüche 1-12, ferner umfassend:
- Aufheben einer Datenübertragung zwischen einem elektronischen medizinischen Protokollsystem und dem Patientenkonto durch den Kontenverwalter auf eine Deaktivierung des Patientenkontos hin, wobei Patientendaten, die zu dem Patientenkonto gehören, in einem Speichermedium gespeichert werden, das zu der Servervorrichtung gehört und/oder funktionell mit dieser gekoppelt ist; und
- Bereitstellen einer Option zum Löschen der Anforderung zur Deaktivieren des Patientenkontos durch den Kontenverwalter.

## Revendications

1. Procédé mis en œuvre par ordinateur pour la gestion de comptes dans un système de gestion de diabète, comprenant :
- la réception, par un gestionnaire de comptes (33), d'une demande pour désactiver un premier compte associé avec un prestataire de services de santé, où le premier compte est associé à une pluralité de comptes de patients et le gestionnaire de comptes est mis en oeuvre sous forme d'exécution d'instructions exécutables par ordinateur sur un processeur d'ordinateur d'un dispositif serveur (18) ;
- le retrait, par le gestionnaire de comptes, de l'accès à des données associées avec le premier compte ;
- l'identification, par le gestionnaire de comptes, de chacun des comptes de patient associé avec le premier compte et la désactivation de chacun des comptes de patient identifiés ;
- la désactivation, par le gestionnaire de comptes, de fonctions de synchronisation de données, les fonctions de synchronisation de données étant conçues pour échanger des données de mesures médicales entre un premier et un deuxième dispositif de stockage par le biais d'un réseau (16), les données échangées faisant partie du premier compte et/ou d'un des comptes de patient identifiés, le premier dispositif de stockage faisant partie et/ou étant couplé opérationnellement au dispositif serveur, le deuxième dispositif de stockage faisant partie d'un dispositif médical portable (28), le dispositif médical portable mesurant et synchronisant de manière répétée des paramètres médicaux d'un patient ;
- l'envoi, par le gestionnaire de comptes, d'une notification électronique à une personne associée avec chacun des comptes de patient identifiés, où la notification électronique avertit la personne que le compte de patient correspondant a été désactivé ; et
- la création, par le gestionnaire de comptes, d'un enregistrement d'audit dans le stockage de données, où l'enregistrement d'audit indique la désactivation du premier compte.

2. Procédé mis en œuvre par ordinateur selon la revendication 1, comprenant en outre l'archivage de données associées avec chacun de la pluralité des comptes de patients pendant un intervalle de temps prédéterminé.

3. Procédé mis en oeuvre par ordinateur selon les revendications 1 ou 2 étant mis en oeuvre dans un système de sécurité pour des données médicales, comprenant :
- le dispositif médical portable (28) pour la mesure de paramètres médicaux étant indicateurs d'un état physiologique d'un mammifère ; et
- le dispositif serveur (18), le dispositif serveur comprenant, et/ou étant couplé de manière fonctionnelle à, un dispositif de stockage, le dispositif serveur étant l'hôte d'un gestionnaire de comptes d'un système de gestion de diabète, où le gestionnaire de comptes est configuré pour gérer des comptes stockés sur ledit dispositif de stockage, lesdits comptes comprenant un premier compte associé avec un prestataire de soins de santé et une pluralité de comptes de patients associés avec ledit premier compte, chaque compte de patient comprenant des données médicales d'un patient respectif ;
- un dispositif mobile (20) étant l'hôte d'un programme d'application (32) étant interopérable avec le dispositif médical portable et avec le gestionnaire de comptes, le dispositif mobile étant connecté avec le dispositif serveur par le biais d'un réseau (16) ;
où le dispositif médical portable est configuré pour mesurer des paramètres médicaux de l'un desdits patients de manière répétée ;
où le programme d'application est configuré pour recevoir de manière répétée et totalement automatisée les paramètres médicaux mesurés à partir dudit dispositif de mesure et est configuré pour transmettre immédiatement les paramètres médicaux mesurés reçus au gestionnaire de comptes pour le stockage desdits paramètres sur le dispositif de stockage du serveur en tant que partie du compte de patient dudit patient, ce par quoi, le dispositif médical portable, le programme d'application et le gestionnaire de comptes sont configurés de telle manière qu'uniquement une seule copie des données médicales mesurées est stockée de manière permanente dans ledit dispositif de stockage et de telle manière qu'une quelconque copie créée de manière temporaire des dites données médicales mesurées est immédiatement effacée du dispositif médical portable, et du dispositif mobile après l'exécution de la transmission ; dans lequel le gestionnaire de comptes est configuré pour exécuter les étapes suivantes :
de réception d'une requête pour désactiver un premier compte ;
de retrait de l'accès aux données associé avec le premier compte ;
d'identification de chacun des comptes de patient associé avec le premier compte et de désactivation de chacun des comptes de patient identifiés ;
d'envoi d'une notification électronique aux patients associés avec chacun des comptes de patient identifiés, où la notification électronique avertit le patient que le compte de patient correspondant a été désactivé ;
de création d'un enregistrement d'audit dans le stockage de données, où l'enregistrement d'audit indique la désactivation du premier compte ; et
d'envoi d'une commande de terminaison aux programmes d'application des patients associés avec chacun des comptes de patient identifié, où les programmes d'application sont configurés de telle manière qu'ils terminent immédiatement la transmission des données de mesure médicale vers le gestionnaire de comptes lors de la réception de la commande de terminaison.

4. Procédé mis en oeuvre par ordinateur selon l'une quelconque des revendications précédentes 1 à 3, comprenant en outre la désactivation, par le gestionnaire de comptes, de fonctions de partage de données, les fonctions de partage de données étant configurées pour échanger des données entre le premier compte et chacun des comptes de patients identifiés, où les données faisant partie du premier compte et des comptes de patients identifiés sont stockées sur un dispositif de stockage qui fait partie et/ou est couplé opérationnellement au dispositif serveur.

5. Procédé mis en oeuvre par ordinateur selon l'une quelconque des revendications précédentes 1 à 4, comprenant en outre l'authentification, par le gestionnaire de comptes, de la demande pour désactiver le premier compte associé avec le prestataire de services de santé, et uniquement désactiver le premier compte dans le cas où la demande a été authentifiée avec succès.

6. Procédé mis en oeuvre par ordinateur selon l'une quelconque des revendications précédentes 1 à 5, comprenant :
- la réception, par le gestionnaire de comptes, d'une demande pour désactiver un compte de patient associé avec le système de gestion de soins du diabète ;
- la fourniture, par le gestionnaire de comptes, d'une notification d'avertissement indiquant que des sessions actives du compte de patient seront terminées ;
- la sollicitation, par le gestionnaire de comptes, d'une confirmation de la demande pour désactiver le compte de patient ;
- la désactivation du compte de patient lors de la réception de la confirmation de la demande pour désactiver le compte de patient ;
- l'envoi, par le gestionnaire de comptes, d'une notification électronique au prestataire de services de santé dont le premier compte est associé avec le compte de patient, où la notification électronique avertit la personne que le compte de patient correspondant a été désactivé ; et
- la création, par le gestionnaire de comptes, d'un enregistrement d'audit dans le stockage de données, où l'enregistrement d'audit indique la désactivation du compte de patient.

7. Procédé mis en oeuvre par ordinateur selon l'une quelconque des revendications précédentes 1 à 6, où deux ou plus de deux comptes de patients sont respectivement associés avec le dispositif médical portable, le procédé comprenant en outre les étapes suivantes devant être exécutées par le gestionnaire de comptes :
- la réception d'une demande pour désactiver tous les dispositifs médicaux portables d'un type de dispositif particulier et/ou d'une version d'un type de dispositif, la demande comprenant un ID de type de dispositif et/ou une version de type de dispositif ;
- l'identification de tous les comptes de patients ayant associés un ID de dispositif d'un dispositif médical portable du type et/ou une version spécifiés dans ladite demande ;
- la désactivation et le dés-enregistrement desdits dispositifs ayant le type et/ou la version spécifiés dans ladite demande dans les comptes de patients identifiés ;
- l'envoi d'une notification électronique aux patients desdits comptes de patients identifiés, où la notification électronique avertit la personne que le dispositif médical portable a été dés-enregistré et/ou a besoin d'une mise à jour usine.

8. Procédé mis en oeuvre par ordinateur selon l'une quelconque des revendications précédentes 1 à 7, où au moins une partie des données est cryptée.

9. Procédé mis en oeuvre par ordinateur selon l'une quelconque des revendications précédentes 1 à 8, où la demande pour désactiver le compte de patient est originaire du fournisseur de soins de santé dont le premier compte est associé avec le compte de patient.

10. Procédé mis en œuvre par ordinateur selon l'une quelconque des revendications précédentes 1 à 9, où la notification électronique est fournie à un patient associé avec le compte de patient.

11. Procédé mis en œuvre par ordinateur selon l'une quelconque des revendications précédentes 1 à 10, comprenant en outre l'authentification, par le gestionnaire de comptes, de la demande pour désactiver le compte de patient avant de désactiver le compte de patient, et de désactiver uniquement le compte de patient dans le cas où la demande a été authentifiée avec succès.

12. Procédé mis en œuvre par ordinateur selon l'une quelconque des revendications précédentes 1 à 11, où le compte de patient est associé avec un dispositif médical portable, et le procédé comprend en outre la désactivation, par le gestionnaire de comptes, de transfert de données entre le dispositif médical portable et ledit compte de patient lors d'une désactivation dudit compte de patient, ce par quoi, des données de patient faisant partie dudit compte de patient sont stockées sur un support de stockage qui fait partie et/ou est couplé opérationnellement au dispositif serveur.

13. Procédé mis en œuvre par ordinateur selon l'une quelconque des revendications précédentes 1 à 12, comprenant en outre :
- la désactivation, par le gestionnaire de comptes, d'un transfert de données entre des enregistrements électroniques médicaux et ledit compte de patient lors d'une désactivation dudit compte de patient, ce par quoi, des données de patient faisant partie dudit compte de patient sont stockées sur un support de stockage qui fait partie et/ou est couplé opérationnellement au dispositif serveur ; et
- la fourniture, par le gestionnaire de comptes, d'une option pour annuler la demande de désactiver le compte de patient.
